# EUROPEAN PATENT APPLICATION

(11) **EP 4 850 596 A1**
(43) Date of publication of application: **09.12.2026**
(21) Application number: 25759897.9
(22) Date of filing: 22.02.2025
(51) Int. Cl.: A61B 5/01, A61B 5/00

(54) **WEARABLE DEVICE, CONTROL METHOD OF SAME, AND PROGRAM**

(30) Priority: 29.02.2024 JP 2024030340
(71) Applicant: Biodata Bank, Inc., Tokyo 150-0031 (JP)
(72) Inventor: SHIOTSU, Takahiro, Tokyo 150-0031 (JP); IZUMOTO, Ryo, Tokyo 150-0031 (JP)
(74) Representative: Pes, Matteo
(86) International application number: PCT/JP2025/006183
(87) International publication number: WO 2025/182846

(57) **Abstract**

To bring a wearable device into an appropriate state according to a situation.

A wearable device (1) comprises a magnetic sensor (31), a switch (32), a power source (33), a measurement part (34), and an MCU (35). The magnetic sensor (31) outputs a low signal when detecting a magnetic force, and outputs a high signal when no longer detecting the magnetic force. In response to the high signal being input from the magnetic sensor (31), the switch (32) connects the power source (33) to the MCU (35) to supply electric power, thereby turning on the wearable device (1). In response to detecting, from a measurement value in the measurement part (34), that a user has worn the wearable device (1) that is in a usable state, the MCU (35) fixes a signal output from the magnetic sensor (31) to the high signal, thereby bringing the wearable device (1) into a constantly usable state.

## Description

### Technical Field

The present invention relates to a wearable device, a control method therefor, and a program, and particularly relates to a wearable device that can be brought into an appropriate state according to a situation, a control method therefor, and a program.

### Background Art

The present applicant has been developing a wearable device that detects heatstroke by detecting that a state in which a deep body temperature is equal to or higher than a predetermined temperature has continued for a predetermined time (see, for example, Patent Literature 1). The description, the scope of claims, and the entire drawings of Patent Literature 1 are incorporated herein by reference.

In the above wearable device, an activation pin was provided on a side face of a device main body, and a user pressed the activation pin to insert the activation pin into the wearable device, thereby turning on an internal switch on a substrate to activate the wearable device.

### Prior art literature

### Patent literature

Patent Literature 1: International Publication No. 2023/218520

### Summary of invention

### Problem to be solved by invention

Once the above activation pin is inserted into the wearable device, it becomes difficult, in terms of the mechanism, to take out the activation pin. Therefore, while there are advantages that the user can be prevented from unintentionally turning off the wearable device and that waterproof performance can be ensured, once the wearable device is turned on, it becomes difficult to turn off the wearable device again. Accordingly, activation confirmation could not be performed in a completed state, and it was necessary to perform complicated quality control. For this reason, the conventional wearable device also had a problem that costs were incurred for the assembly and the inspection thereof.

In addition, there were also problems that the user forgot to press the activation pin so that the wearable device was not activated, or that the insertion of the activation pin was not performed well so that the waterproof performance could be impaired.

The present invention has been made to solve the above problems, and an object thereof is to provide a wearable device that can be brought into an appropriate state according to a situation, a control method therefor, and a program.

### Means For Solving Problem

To achieve the above purpose, a wearable device (1) of a first aspect of the present invention is a wearable device (1) wearable by a user, in which the wearable device (1) comprises: a signal output part (31) that, in response to a first condition being satisfied, changes a signal to be output from a signal of a first level to a signal of a second level different from the first level, thereby bringing the wearable device (1) into a usable state; and a controller part (35) that, in response to a second condition different from the first condition being satisfied, brings the wearable device (1) into a constantly usable state.

In the above wearable device (1), the controller part (35) may, when detecting that the user has worn the wearable device (1) that is in the usable state, determine that the second condition is satisfied, and may bring the wearable device (1) into the constantly usable state.

The above wearable device (1) may further comprise a fixing switch (11), in which, when the user turns on the fixing switch (11) of the wearable device (1) that is in the usable state, the controller part (35) may determine that the second condition is satisfied, and may bring the wearable device (1) into the constantly usable state.

In the above wearable device (1), the signal output part (31) may be a magnetic sensor that outputs the signal of the first level when detecting a magnetic force, and that outputs the signal of the second level upon determining that the first condition is satisfied when no longer detecting the magnetic force.

The above wearable device (1) may further comprise a seal (5) having a magnetic force and affixed near the signal output part (31), in which the signal output part (31) may output the signal of the second level when the seal (5) is peeled off and the magnetic force from the seal (5) is no longer detected.

The wearable device (1) may be inserted into a package (6), the package (6) may have a portion having a magnetic force near the signal output part (31) of the inserted wearable device (1), and the signal output part (31) may output the signal of the second level when the wearable device (1) is taken out from the package (6) and the magnetic force from the package (6) is no longer detected.

The above wearable device (1) may further comprise: a power source (33) that supplies electric power; and a switch (32, 112) that, in response to the second signal being input from the signal output part (31), connects the power source (33) to the controller part (35) to supply the electric power, thereby turns on and brings the wearable device (1) into the usable state.

In the above wearable device (1), the controller part (35) may determine whether or not the signal input from the signal output part (31) is the signal of the second level, and, when determining that the signal input from the signal output part (31) is the signal of the second level, may bring the wearable device (1) into the constantly usable state in response to the second condition being satisfied.

In the above wearable device (1), in response to the second condition being satisfied, the controller part (35) may fix the signal output from the signal output part (31) to the signal of the second level, thereby bringing the wearable device (1) that is in the usable state into the constantly usable state.

The above wearable device (1) may further comprise a measurement part (34) that measures biological information of the user wearing the wearable device (1), in which the controller part (35) may detect, from a measurement value in the measurement part (34), that the user is wearing the wearable device (1).

To achieve the above purpose, a control method according to a second aspect of the present invention is a control method for a wearable device (1) wearable by a user, and comprises: in response to a first condition being satisfied, changing a signal to be output from a signal of a first level to a signal of a second level different from the first level by a signal output part (31), thereby bringing the wearable device (1) into a usable state; and, in response to a second condition different from the first condition being satisfied, bringing the wearable device (1) into a constantly usable state by a controller part (35).

To achieve the above purpose, a program according to a third aspect of the present invention causes a computer of a wearable device (1), which is wearable by a user and changes a signal to be output from a signal of a first level to a signal of a second level different from the first level in response to a first condition being satisfied, to execute: a procedure of determining whether or not a signal input from a signal output part (31) is the signal of the second level; and a procedure of, when it is determined that the signal input from the signal output part (31) is the signal of the second level, bringing the wearable device (1) into a constantly usable state in response to a second condition different from the first condition being satisfied.

### Advantageous Effect of Invention

According to the present invention, it is possible to provide the wearable device that can be brought into an appropriate state according to a situation, the control method therefor, and the program.

### Brief Description of Drawings

Fig. 1 is an external perspective view of a configuration example of a wearable device according to the present embodiment.
Fig. 2 is a diagram illustrating a circuit configuration of a device main body according to the present embodiment.
Fig. 3 includes diagrams for explaining a function of a magnetic sensor.
Fig. 4 (a) is an explanatory diagram of an operation example of the device main body before worn by a user and when a magnetic force is not detected, and (b) is an explanatory diagram of an operation example of the device main body before worn by the use and when the magnetic force is detected.
Fig. 5 is a view of an example of a state of the wearable device at the time of shipment.
Fig. 6 is a view of another example of the state of the wearable device at the time of shipment.
Fig. 7 is an explanatory diagram of an operation example of the device main body after worn by the user.
Fig. 8 is a diagram illustrating a circuit configuration of a device main body according to modified embodiment 1.
Fig. 9 is a flowchart illustrating an example of control processing executed by the device main body according to modified embodiment 1.
Fig. 10 (a) is a front view of a configuration example of a device main body according to modified embodiment 2, and (b) is a side perspective view of the configuration example of the device main body according to modified embodiment 2.
Fig. 11 is a diagram illustrating a circuit configuration of the device main body according to modified embodiment 2.
Fig. 12 is a sequence diagram illustrating an operation example of the device main body according to modified embodiment 2.

### Embodiment For Practicing Invention

Hereinafter, an embodiment for practicing the present invention will be described.

First, a configuration of a wearable device according to the embodiment of the present invention will be described with reference to the drawings.

The wearable device according to the present embodiment is wearable by a user, and includes, for example, an electronic apparatus of a wristwatch such as a smart watch, and the like.

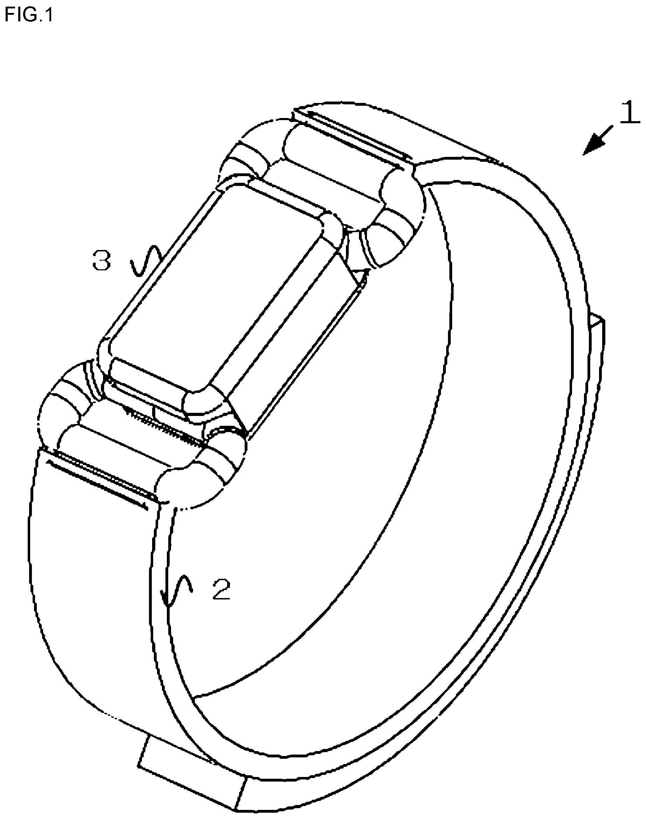

Fig. 1 is an external perspective view of a configuration example of the wearable device according to the present embodiment.

As illustrated in Fig. 1, the wearable device 1 includes a band 2 and a device main body 3. In the present embodiment, when the wearable device 1 is worn, a face of the device main body 3 that contacts a wrist of the user is referred to as a rear face, a face on the opposite side thereof (a face on which a touch screen is provided) is referred to as a front face, joint faces between the band 2 and the device main body 3 are referred to as an upper face and a lower face, and the remaining faces are referred to as side faces.

The band 2 is a member for wearing the wearable device 1. The user can wear the wearable device 1 by wrapping the band 2 around the vicinity of the wrist such that the rear face of the device main body 3 faces inward.

The device main body 3 includes an electronic apparatus, which includes various sensors, the touch screen, a wireless communication device, an operation button, a speaker, a Micro Controller Unit (MCU), and the like. The device main body 3 measures, with the sensors, biological information such as a deep body temperature and a blood pressure of the user under control by the MCU, and displays the biological information on the touch screen or wirelessly transmits the biological information to an external computer such as a smartphone.

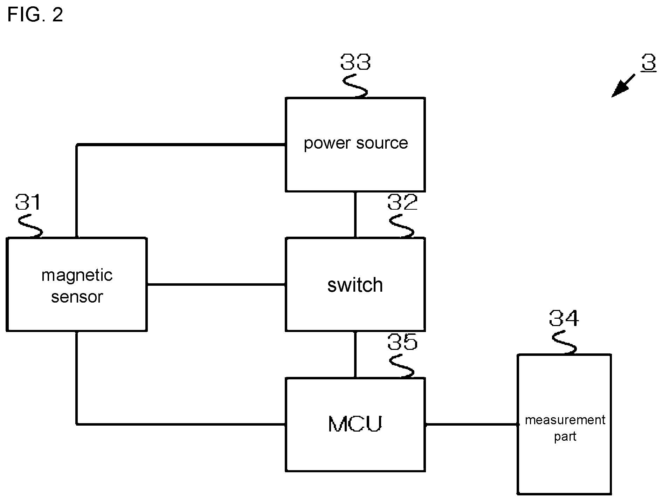

Fig. 2 is a diagram illustrating a circuit configuration of the device main body according to the present embodiment.

As illustrated in Fig. 2, the device main body 3 includes a magnetic sensor (signal output part) 31, a switch 32, a power source 33, a measurement part 34, and an MCU (controller part) 35.

The magnetic sensor 31 includes, for example, a material such as a Hall element, a magnetic impedance element, or a coil.

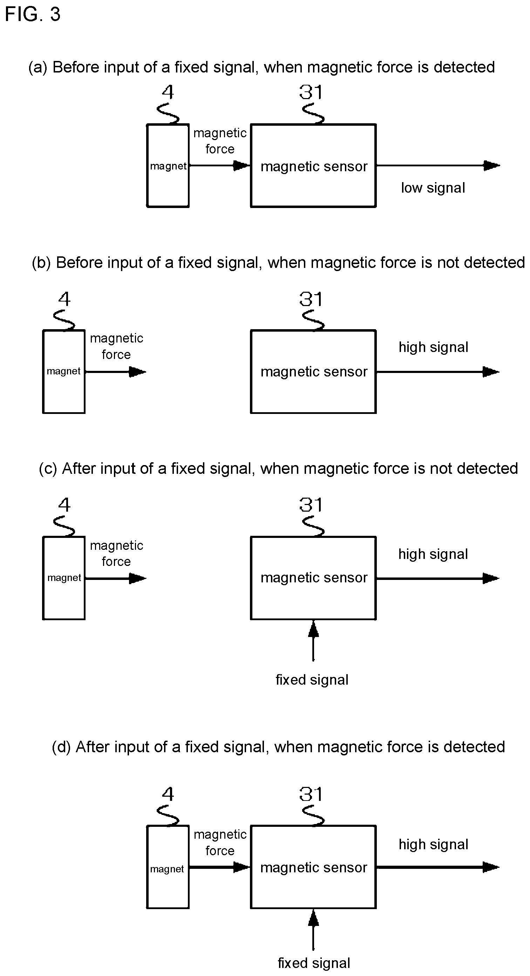

Fig. 3 includes diagrams for explaining a function of the magnetic sensor.

As illustrated in Fig. 3(a), while a magnet 4 is nearby and the magnetic sensor 31 is detecting a magnetic force from the magnet 4, the magnetic sensor 31 outputs a low-level (first level) signal (hereinafter referred to as a "low signal"), and, as illustrated in Fig. 3(b), in response to that the magnet 4 has moved away and the magnetic force is no longer detected (a first condition is satisfied), the magnetic sensor 31 outputs a high-level (second level) signal (hereinafter referred to as a "high signal").

When a fixing signal is supplied from the MCU 35, the magnetic sensor 31 fixes the signal to be output.

Specifically, as illustrated in Fig. 3(c), when the fixing signal is input from the MCU 35 while the magnetic sensor 31 is outputting the high signal, the signal to be output is fixed to the high signal. Thereafter, as illustrated in Fig. 3(d), even when the magnet 4 approaches and the magnetic force comes to be detected, the signal to be output does not become the low signal but remains the high signal.

The switch 32 is formed by, for example, a semiconductor switch such as a Metal-Oxide-Semiconductor Field-Effect Transistor (MOSFET), a bipolar transistor, an Insulated Gate Bipolar Transistor (IGBT), a Gallium Nitride (GaN) transistor, or a Silicon Carbide (SiC) transistor, a relay switch, a regulator, or the like.

While the low signal is input from the magnetic sensor 31, that is, while the magnet 4 is located near the magnetic sensor 31 and the magnetic sensor 31 is detecting the magnetic force from the magnet 4, the switch 32 does not connect the power source 33 to the MCU 35.

Thereafter, when the high signal is input from the magnetic sensor 31, that is, when the magnet 4 moves away from the magnetic sensor 31 and the magnetic sensor 31 no longer detects the magnetic force, the switch 32 connects the power source 33 to the MCU 35.

The power source 33 includes, for example, a battery such as a general-purpose primary battery or secondary battery, or the like. The power source 33 is connected to the MCU 35 by the switch 32 and supplies electric power to the MCU 35. As a result, the wearable device 1 is activated and turned on.

In this way, when the magnet 4 moves away from the magnetic sensor 31 and the magnetic sensor 31 no longer detects the magnetic force, the signal output from the magnetic sensor 31 becomes the high signal, the power source 33 and the MCU 35 are connected by the switch 32, and the wearable device 1 is turned on. That is, when the wearable device 1 is on, the signal output from the magnetic sensor 31 is the high signal.

The measurement part 34 is a sensor unit that includes, for example: a general-purpose temperature sensor formed by a thermistor whose resistance value changes depending on temperature, or the like; a general-purpose heat flux sensor formed by a Peltier element or the like; and the like, and measures biological information such as a temperature of a body surface of the user wearing the wearable device 1 and a heat flow from a deep position such as a brain or an organ of the user.

The MCU 35 is driven by receiving the electric power supplied from the power source 33, uses a Random Access Memory (RAM) as a working memory, and appropriately executes various programs and the like stored in a Read Only Memory (ROM) and a storage part, whereby controlling various operations of the device main body 3.

In the present embodiment, the MCU 35 determines whether the user has worn the wearable device 1, from the temperature, the heat flow, and the like (measurement values) measured by the measurement part 34. When determining that the user has worn the wearable device 1, the MCU 35 supplies, to the magnetic sensor 31, the fixing signal for fixing the signal output from the magnetic sensor 31.

When the fixing signal is supplied from the MCU 35 to the magnetic sensor 31, the wearable device 1 is naturally on, and therefore the signal output from the magnetic sensor 31 is always the high signal. For this reason, when the signal output from the magnetic sensor 31 is fixed by the fixing signal, the signal is always fixed to the high signal. As a result, the wearable device 1 is constantly on until the electric power is no longer supplied from the power source 33 due to battery exhaustion or the like, is no longer turned off by environmental noise such as the user unintentionally approaching a magnetized object such as the magnet 4, and is brought into a constantly usable state.

After the wearable device 1 is brought into the constantly usable state, the MCU 35 executes measurement processing. Specifically, the MCU 35 measures the deep body temperature of the user based on the temperature, the heat flow, and the like measured by the measurement part 34, or measures the blood pressure of the user based on a measurement value of an optical sensor provided in the measurement part 34, and displays the biological information such as the deep body temperature and the blood pressure of the user obtained by the measurement on the touch screen or wirelessly transmits the biological information to an external computer such as a smartphone.

Next, an operation of the wearable device having the above configuration will be described with reference to the drawings.

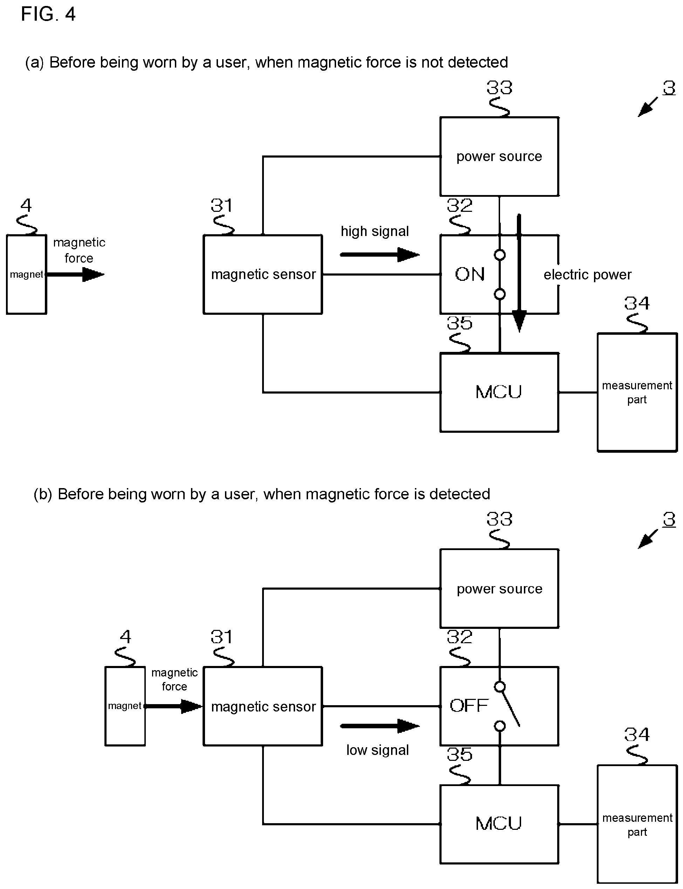

Fig. 4(a) is an explanatory diagram of an operation example of the device main body before worn by the user and when the magnetic force is not detected, and Fig. 4(b) is an explanatory diagram of the operation example of the device main body before worn by the user and when the magnetic force is detected.

As illustrated in Fig. 4(a), after the completion of the wearable device 1 and before shipment, the user has not yet worn the wearable device 1, so that the signal output from the magnetic sensor 31 is not fixed. In this case, if the magnet 4 is so far away that the magnetic sensor 31 cannot detect the magnetic force thereof, the signal output from the magnetic sensor 31 becomes the high signal, and the wearable device 1 is turned on.

On the other hand, as illustrated in Fig. 4(b), if the magnet 4 is brought so close that the magnetic sensor 31 can detect the magnetic force thereof, the signal output from the magnetic sensor 31 becomes the low signal, and the wearable device 1 is turned off.

As a result, it is possible to provide an inspection process in which, at a factory after the completion of the wearable device 1 and before shipment, the magnet 4 is moved away or brought close to confirm check whether or not the wearable device 1 is normally switched on/off and to confirm whether or not the wearable device 1 is normally activated.

In this way, unlike a conventional wearable device that is turned on by pressing an activation pin that is difficult to pull out, the wearable device 1 can be inspected in a completed state as to whether it is normally activated.

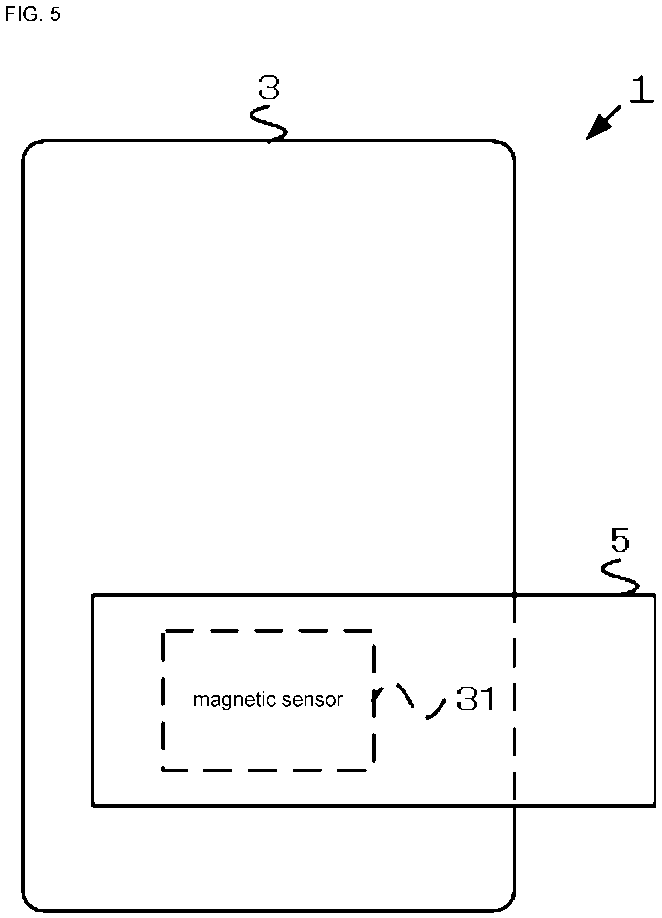

Fig. 5 is a view of an example of the state of the wearable device at the time of shipment.

Then, when it is confirmed in the above inspection process that the wearable device 1 is normally activated, as illustrated in Fig. 5, a seal (hereinafter referred to as a "magnet seal") 5 having a magnetic force in the same manner as the magnet 4 is affixed near the magnetic sensor 31 of the device main body 3, and the wearable device 1 is turned off. Thereafter, the wearable device 1 is shipped. In this case, when the user obtains the wearable device 1 and peels off the magnet seal 5 at the time of starting to use, the magnetic sensor 31 of the wearable device 1 can no longer detect the magnetic force from the magnet seal 5, and therefore the wearable device 1 is turned on and brought into the usable state.

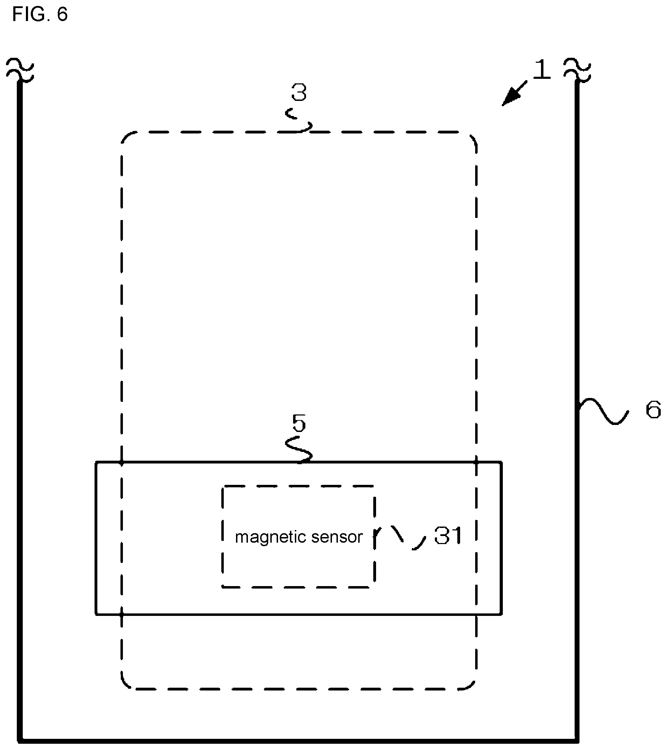

Fig. 6 is a view of another example of the state of the wearable device at the time of shipment.

Alternatively, as illustrated in Fig. 6, the wearable device 1 is shipped after being inserted into a package 6, to which the magnet seal 5 having the magnetic force is affixed at a portion that comes near the magnetic sensor 31 when the wearable device 1 is inserted, and then turned off. In this case, when the user obtains and takes out the wearable device 1 from the package 6, to which the magnet seal 5 is affixed, at the time of starting to use, the magnetic sensor 31 of the wearable device 1 can no longer detect the magnetic force from the package 6, and therefore the wearable device 1 is turned on and brought into the usable state.

In this way, the user can turn on (power on) the wearable device 1 in the course of a natural action such as peeling off the magnet seal 5 or taking the wearable device 1 out of the package 6 to which the magnet seal 5 is affixed, at the time of starting to use after obtaining the wearable device 1. Therefore, the user no longer forgets to activate the wearable device 1.

In addition, the wearable device 1 can be turned on (powered on) by an external magnetic switch function to move away the object having the magnetic force, such as the magnet seal 5 or the package 6. Thus, unlike the conventional wearable device, it is no longer necessary to provide the activation pin. As a result, the wearable device 1 can ensure waterproof performance regardless of whether it is before or after use by the user, and therefore the waterproof performance can be improved as compared with the conventional wearable device.

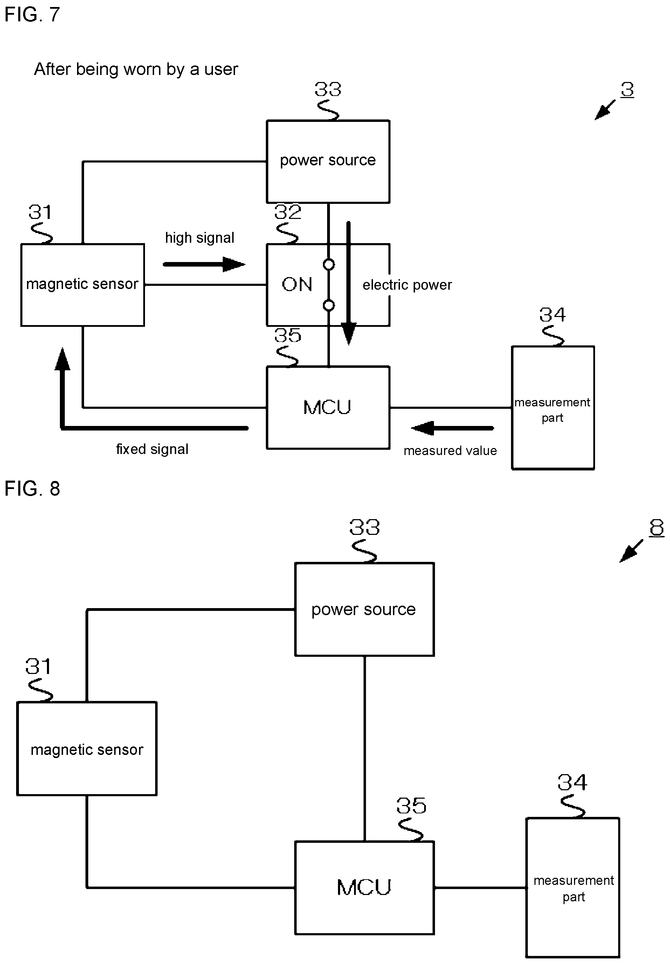

Fig. 7 is an explanatory diagram of an operation example of the device main body after worn by the user.

Thereafter, in response to detecting that the user has worn the wearable device 1 that is in the usable state (a second condition being satisfied), the MCU 35 supplies the fixing signal to the magnetic sensor 31 as illustrated in Fig. 7, and fixes the signal output from the magnetic sensor 31 to the high signal.

The wearable device 1 can be brought into the constantly usable state by such an internal circuit configuration. As a result, the wearable device 1 becomes able to continue to measure the biological information, such as the deep body temperature and the blood pressure of the user, without being turned off by the environmental noise, such as the user unintentionally approaching the magnetized object such as the magnet 4, until the electric power is no longer supplied from the power source 33 due to the battery exhaustion or the like, and the user experience (User Experience; UX) is improved.

As described above, the wearable device 1 according to the present embodiment is wearable by the user, and comprises the magnetic sensor (signal output part) 31, the switch 32, the power source 33 that supplies the electric power, the measurement part 34 that measures the biological information of the user wearing the wearable device 1, and the MCU (controller part) 35.

The magnetic sensor 31 outputs the low signal (the signal of the first level) when detecting the magnetic force, and outputs the high signal (the signal of the second level different from the first level) when no longer detecting the magnetic force. That is, in response to the magnetic force being no longer detected (the first condition being satisfied), the magnetic sensor 31 changes the signal to be output from the low signal to the high signal. As a result, the magnetic sensor 31 turns on the wearable device 1 and brings it into the usable state.

Specifically, in response to the high signal being input from the magnetic sensor 31, the switch 32 connects the power source 33 to the MCU 35 to supply the electric power. In this way, the wearable device 1 is turned on and brought into the usable state.

As an example, at the time of factory shipment, the wearable device 1 further comprises the seal (magnet seal) 5 having the magnetic force and affixed near the magnetic sensor 31. That is, at the time of factory shipment, the wearable device 1 is off. When the user obtains the wearable device 1 and thereafter starts using the wearable device 1, the magnet seal 5 is peeled off. Then, the magnetic sensor 31 outputs the high signal when no longer detecting the magnetic force from the magnet seal 5. As a result, the wearable device 1 is turned on and brought into the usable state.

As another example, at the time of factory shipment, the wearable device 1 is inserted into the package 6. The package 6 has the portion having the magnetic force (the portion to which the magnet seal 5 is affixed) near the magnetic sensor 31 of the inserted wearable device 1. That is, at the time of factory shipment, the wearable device 1 is off. When the user obtains the wearable device 1 and thereafter starts using the wearable device 1, the wearable device 1 is taken out from the package 6. Then, the magnetic sensor 31 outputs the high signal when no longer detecting the magnetic force from the package 6. As a result, the wearable device 1 is turned on and brought into the usable state.

Then, in response to detecting that the user has worn the wearable device 1 that is in the usable state (the second condition being satisfied), the MCU 35 fixes the signal output from the magnetic sensor 31 to the high signal, thereby constantly turning on the wearable device 1 to bring the wearable device 1 into the usable state. Specifically, the MCU 35 detects, from the measurement value in the measurement part 34, that the user is wearing the wearable device 1.

In this way, since the wearable device 1 can be switched on/off until it is detected that the user has worn the wearable device 1. Thus, after the completion and before shipment, the inspection can be performed to confirm whether the wearable device 1 is normally activated at a factory. Meanwhile, since the wearable device 1 is fixed to be constantly on once it is detected that the user has worn the wearable device 1, the wearable device 1 becomes able to continue to measure the biological information, such as the deep body temperature and the blood pressure of the user, without being turned off by the environmental noise, such as the user unintentionally approaching the magnetized object such as the magnet 4, until the electric power is no longer supplied from the power source 33 due to the battery exhaustion or the like.

As a result, the wearable device 1 according to the present embodiment can be brought into an appropriate state according to a situation.

The present invention is not limited to the above embodiment, and various modifications and applications are possible. Hereinafter, modified aspects of the above embodiment applicable to the present invention will be described.

### [Modified Embodiment 1]

In the above embodiment, the device main body 3 has been described as comprising the switch 32. However, the present invention should not be limited thereto, and the device main body may not comprise the switch 32.

Fig. 8 is a diagram illustrating a circuit configuration of a device main body according to modified embodiment 1.

The same signs are given to the same components as those of the device main body 3 according to the above embodiment, and the description thereof is omitted.

As illustrated in Fig. 8, a device main body 8 according to the present modified embodiment comprises the magnetic sensor (signal output part) 31, the power source 33, the measurement part 34, and the MCU (controller part) 35.

In the present modified embodiment, unlike the above embodiment, the power source 33 is directly connected to the MCU 35 and constantly supplies the electric power to the MCU 35.

In the present modified embodiment, the MCU 35 changes the state of the wearable device 1 in accordance with the signal input from the magnetic sensor 31. Specifically, when the signal input from the magnetic sensor 31 is the low signal, the MCU 35 brings the wearable device 1 into a power saving state (sleep state) in which power consumption is suppressed, and, when the signal is the high signal, the MCU 35 brings the wearable device 1 into a normal operating state (the usable state).

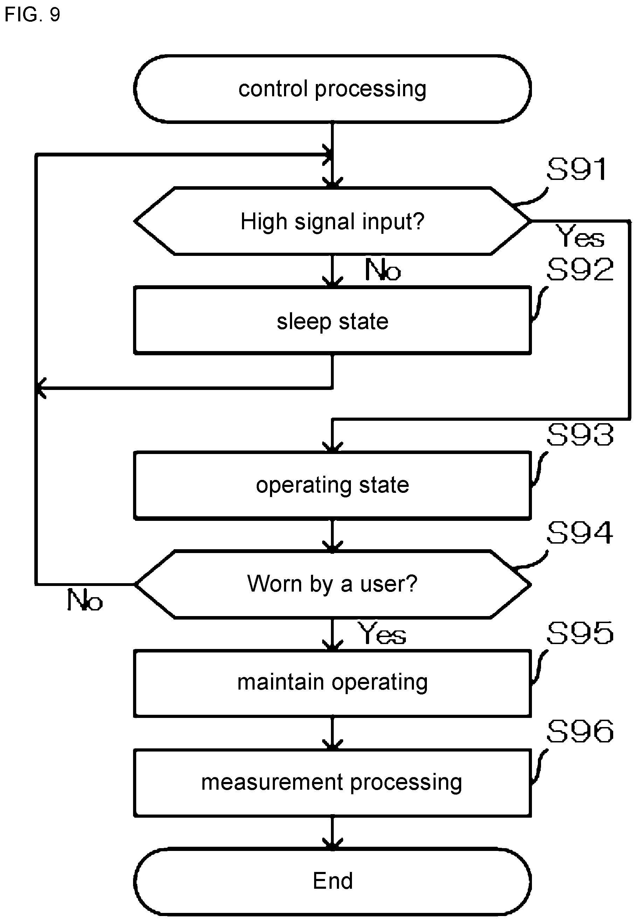

Fig. 9 is a flowchart illustrating an example of control processing executed by the device main body according to modified embodiment 1.

In the control processing illustrated in Fig. 9, the MCU 35 determines whether the signal input from the magnetic sensor 31 is the low signal or the high signal (Step S91).

If determining that the signal input from the magnetic sensor 31 is the low signal (Step S91; No), the MCU 35 brings the wearable device 1 into the power saving state (sleep state) in which the power consumption is suppressed (Step S92), and the processing returns to Step S91.

On the other hand, if determining that the signal input from the magnetic sensor 31 is the high signal (Step S91; Yes), the MCU 35 brings the wearable device 1 into the normal operating state (the usable state) (Step S93).

After the wearable device 1 is brought into the usable state, the MCU 35 determines whether or not the user has worn the wearable device 1, from the temperature, the heat flow, and the like (measurement values) measured by the measurement part 34 (Step S94).

If determining that the user is not wearing the wearable device 1 (Step S94; No), the processing returns to Step S91.

On the other hand, if determining that the user has worn the wearable device 1 (Step S94; Yes), the MCU 35 maintains the usable state regardless of the signal output from the magnetic sensor 31 (Step S95).

Since the MCU 35 maintains the usable state regardless of the signal output from the magnetic sensor 31, the wearable device 1 is brought into the constantly usable state. In this way, in the case where it is detected even once that the user has worn the wearable device 1 while the wearable device 1 is in the usable state, the wearable device 1 is brought into the constantly usable state without entering the sleep state until the electric power is no longer supplied from the power source 33 due to the battery exhaustion or the like.

After the wearable device 1 is brought into the constantly usable state, the MCU 35 executes measurement processing (Step S96). Specifically, the MCU 35 measures the deep body temperature of the user based on the temperature, the heat flow, and the like measured by the measurement part 34, or measures the blood pressure of the user based on the measurement value of the optical sensor provided in the measurement part 34, and displays the biological information, such as the deep body temperature and the blood pressure of the user obtained by the measurement, on the touch screen or wirelessly transmits the biological information to an external computer such as a smartphone.

As a result, the wearable device 1 according to the present modified embodiment can exhibit operational effects similar to those of the wearable device 1 according to the above embodiment, and can be brought into an appropriate state according to a situation.

### [Modified Embodiment 2]

In the above embodiment, it has been described that the second condition is satisfied when it is detected that the user has worn the wearable device 1 that is in the usable state. However, the present invention should not be limited thereto, and, for example, the second condition may be satisfied when the user turns on a fixing switch provided on the wearable device 1.

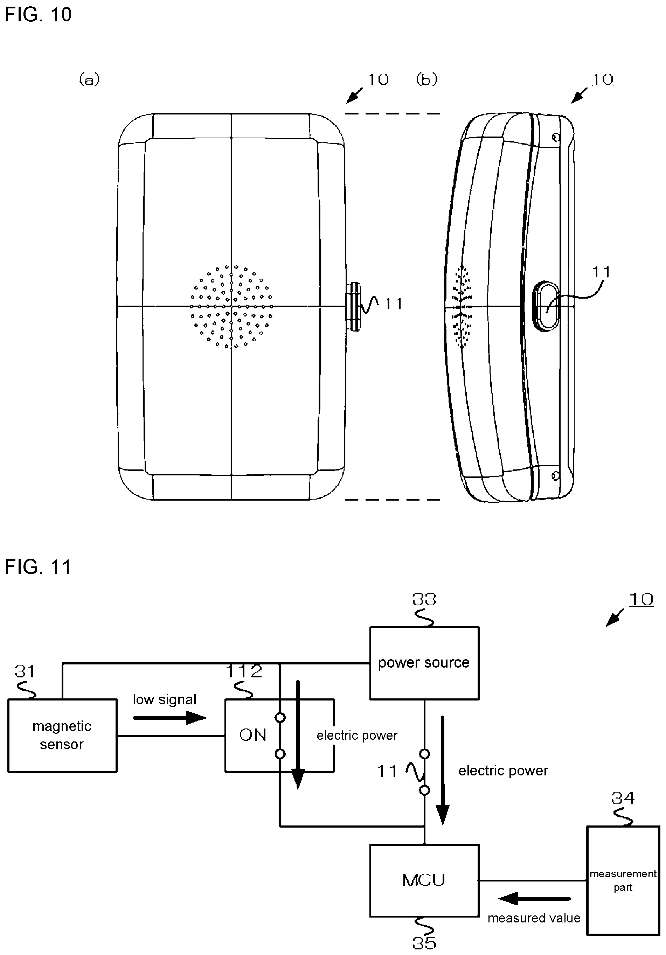

Fig. 10(a) is a front view of a configuration example of a device main body according to modified embodiment 2, and Fig. 10(b) is a side perspective view of the configuration example of the device main body according to modified embodiment 2.

The same signs are given to the same components as those of the wearable device 1 according to the above embodiment and modified embodiment 1, and the description thereof is omitted.

As illustrated in Figs. 10(a) and (b), a device main body 10 according to the present modified embodiment comprises a fixing switch 11 on a side face thereof.

Fig. 11 is a diagram illustrating a circuit configuration of the device main body according to modified embodiment 2.

As illustrated in Fig. 11, the device main body 10 comprises the fixing switch 11, the magnetic sensor (signal output part) 31, a switch 112, the power source 33, the measurement part 34, and the MCU (controller part) 35.

Unlike the switch 32, while the high signal is input from the magnetic sensor 31, that is, while the magnet 4 is far from the magnetic sensor 31 and the magnetic sensor 31 is not detecting the magnetic force, the switch 112 does not connect the power source 33 to the MCU 35.

Thereafter, when the low signal is input from the magnetic sensor 31, that is, when the magnet 4 approaches the magnetic sensor 31 and the magnetic sensor 31 detects the magnetic force, the switch 112 connects the power source 33 to the MCU 35.

Until the user turns on the fixing switch 11, the power source 33 and the MCU 35 are not connected. Thus, the wearable device 1 can be switched on/off by bringing the magnet 4 close or moving the magnet 4 away. When the user turns on the fixing switch 11, the power source 33 and the MCU 35 are connected. Thus, the wearable device 1 remains on even when the magnet 4 is moved away or brought close.

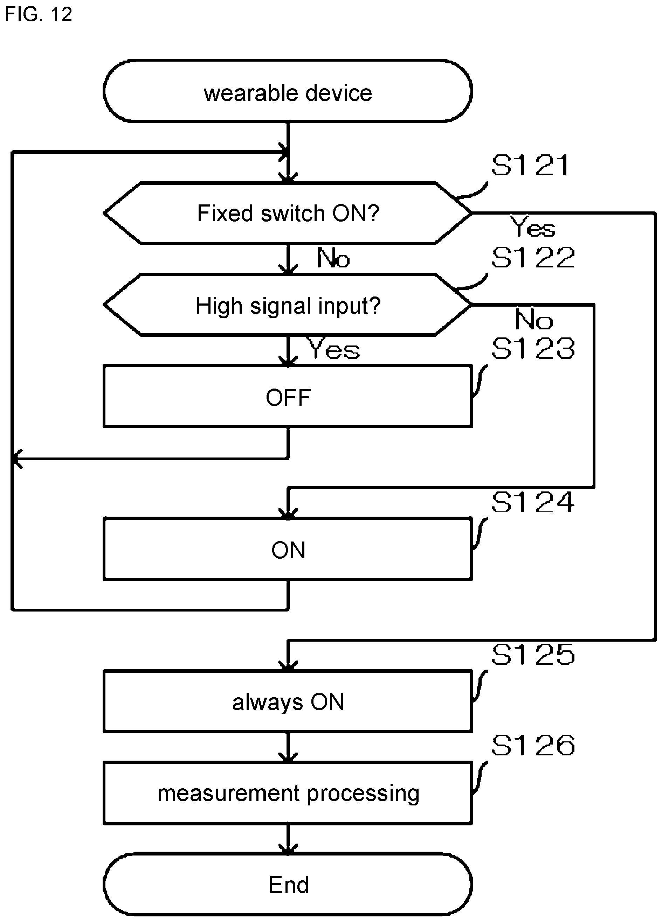

Fig. 12 is a sequence diagram illustrating an operation example of the device main body according to modified embodiment 2.

As illustrated in Fig. 12, until the user turns on the fixing switch 11 of the wearable device 1 (Step S121; No), the power source 33 and the MCU 35 are not connected. Thus, if the magnet 4 is moved so far away that the magnetic sensor 31 cannot detect the magnetic force thereof, the signal output from the magnetic sensor 31 becomes the high signal (Step S122; Yes), and the wearable device 1 is turned off (Step S123). On the other hand, if the magnet 4 is so close that the magnetic sensor 31 can detect the magnetic force thereof, the signal output from the magnetic sensor 31 becomes the low signal (Step S122; No), and the wearable device 1 is turned on (Step S124).

When the user turns on the fixing switch 11 of the wearable device 1 (Step S121; Yes), the power source 33 and the MCU 35 are connected, and thus the wearable device 1 is constantly turned on and brought into the usable state (Step S125). Then, after the wearable device 1 is brought into the constantly usable state, the MCU 35 executes measurement processing (Step S126).

In the present modified embodiment, when the user turns on the fixing switch 11 of the wearable device 1 that is in the usable state, the MCU 35 determines that the second condition is satisfied, and thus it is only necessary to connect the power source 33 and the MCU 35 and constantly turn on and bring the wearable device 1 into the usable state.

As a result, the wearable device 1 according to the present modified embodiment can exhibit operational effects similar to those of the wearable device 1 according to the above embodiment and modified embodiment 1, and can be brought into an appropriate state according to a situation.

In the above embodiment and modified embodiments 1 and 2, it has been described that the signal output part is the magnetic sensor 31 that outputs the signal of the first level when detecting the magnetic force and that outputs the signal of the second level different from the first level when no longer detecting the magnetic force. However, the present invention should not be limited thereto, and the signal output part can be changed appropriately as long as the signal output part changes the signal to be output from the signal of the first level to the signal of the second level different from the first level in response to the first condition being satisfied.

In addition, in the above embodiment and modified embodiments 1 and 2, it has been described that the magnetic sensor 31 outputs the low signal when detecting the magnetic force and outputs the high signal when no longer detecting the magnetic force. However, the present invention should not be limited thereto, and any sensor is acceptable as long as the sensor outputs signals of different levels when not detecting the magnetic force and when detecting the magnetic force. For example, the magnetic sensor 31 may output the high signal when detecting the magnetic force and output the low signal when no longer detecting the magnetic force. In this case, in the above embodiment, the switch 32 only needs to turn on the wearable device 1 by connecting the power source 33 to the MCU 35 to supply the electric power when the signal input from the magnetic sensor 31 is the low signal. In addition, in modified embodiment 1 described above, the MCU 35 only needs to bring the wearable device 1 into the normal operating state (usable state) when determining that the signal input from the magnetic sensor 31 is the low signal. In modified embodiment 2 described above, the switch 112 only needs to turn on the wearable device 1 by connecting the power source 33 to the MCU 35 to supply the electric power when the signal input from the magnetic sensor 31 is the high signal.

In the above embodiment and modified embodiments, the program executed by the MCU has been described as being stored in advance in the ROM, the storage part, or the like. However, the present invention should not be limited thereto. A program for executing the above-described processing may be applied to an existing general-purpose computer, and the computer may be made to function as the device main body 3 according to the above embodiment and modified embodiments.

Any appropriate method for providing such a program can be used. For example, the program may be stored in a computer-readable recording medium (a flexible disk, a Compact Disc (CD)-ROM, a Digital Versatile Disc (DVD)-ROM, or the like) and distributed, or the program may be stored in a storage on a network such as the Internet and provided by being downloaded therefrom.

Furthermore, when the above processing is executed by sharing between an Operating System (OS) and an application program, or by cooperation between the OS and the application program, only the application program may be stored in the recording medium or the storage. Alternatively, it is also possible to superimpose the program on a carrier wave and distribute the program via the network. For example, the program may be posted on a bulletin board system (BBS) on the network, and the program may be distributed via the network. Then, this program may be started and executed under the control of the OS in the same manner as other application programs, so that the above processing can be executed.

The present invention allows various embodiments and modifications without departing from the spirit and scope in wider meaning of the present invention. In addition, the embodiments described above are provided for explaining examples of the present invention, and do not limit the scope of the present invention.

This application is based on Japanese Patent Application No. 2024-030340 filed on February 29, 2024. The description, the scope of claims, and the entire drawings of Japanese Patent Application No. 2024-030340 are incorporated herein by reference.

### Description Of Signs

wearable device
band
device main body
magnet
magnet seal
package
fixing switch
magnetic sensor (signal output part)
switch
power source
measurement part
MCU (controller part)

## Claims

1. A wearable device (1) wearable by a user, the wearable device (1) comprising:
a signal output part (31) that, in response to a first condition being satisfied, changes a signal to be output from a signal of a first level to a signal of a second level different from the first level, thereby bringing the wearable device (1) into a usable state; and
a controller part (35) that, in response to a second condition different from the first condition being satisfied, brings the wearable device (1) into a constantly usable state.

2. The wearable device (1) according to claim 1, wherein
the controller part (35), when detecting that the user has worn the wearable device (1) that is in the usable state, determines that the second condition is satisfied, and brings the wearable device (1) into the constantly usable state.

3. The wearable device (1) according to claim 1 further comprising:
a fixing switch (11), wherein
when the user turns on the fixing switch (11) of the wearable device (1) that is in the usable state, the controller part (35) determines that the second condition is satisfied, and brings the wearable device (1) into the constantly usable state.

4. The wearable device (1) according to claim 1, wherein
the signal output part (31) is a magnetic sensor that outputs the signal of the first level when detecting a magnetic force, and that outputs the signal of the second level upon determining that the first condition is satisfied when no longer detecting the magnetic force.

5. The wearable device (1) according to claim 4 further comprising:
a seal (5) having a magnetic force and affixed near the signal output part (31), wherein
the signal output part (31) outputs the signal of the second level when the seal (5) is peeled off and the magnetic force from the seal (5) is no longer detected.

6. The wearable device (1) according to claim 4, wherein
the wearable device (1) is inserted into a package (6),
the package (6) has a portion having a magnetic force near the signal output part (31) of the inserted wearable device (1), and
the signal output part (31) outputs the signal of the second level when the wearable device (1) is taken out from the package (6) and the magnetic force from the package (6) is no longer detected.

7. The wearable device (1) according to claim 1 further comprising:
a power source (33) that supplies electric power; and
a switch (32, 112) that, in response to the second signal being input from the signal output part (31), connects the power source (33) to the controller part (35) to supply the electric power, and thereby turns on and brings the wearable device (1) into the usable state.

8. The wearable device (1) according to claim 1, wherein
the controller part (35)
determines whether or not the signal input from the signal output part (31) is the signal of the second level, and
when determining that the signal input from the signal output part (31) is the signal of the second level, brings the wearable device (1) into the constantly usable state in response to the second condition being satisfied.

9. The wearable device (1) according to claim 1, wherein
in response to the second condition being satisfied, the controller part (35) fixes the signal output from the signal output part (31) to the signal of the second level, thereby bringing the wearable device (1) that is in the usable state into the constantly usable state.

10. The wearable device (1) according to claim 2 further comprising:
a measurement part (34) that measures biological information of the user wearing the wearable device (1), wherein
the controller part (35) detects, from a measurement value in the measurement part (34), that the user is wearing the wearable device (1).

11. A control method for a wearable device (1) wearable by a user, the control method comprising:
in response to a first condition being satisfied, changing a signal to be output from a signal of a first level to a signal of a second level different from the first level by a signal output part (31), thereby bringing the wearable device (1) into a usable state; and
in response to a second condition different from the first condition being satisfied, bringing the wearable device (1) into a constantly usable state by a controller part (35).

12. A program for causing a computer of a wearable device (1) wearable by a user, the wearable device (1) changing a signal to be output from a signal of a first level to a signal of a second level different from the first level in response to a first condition being satisfied, the program for causing the computer to execute:
a procedure of determining whether or not a signal input from a signal output part (31) is the signal of the second level; and
a procedure of, when it is determined that the signal input from the signal output part (31) is the signal of the second level, bringing the wearable device (1) into a constantly usable state in response to a second condition different from the first condition being satisfied.
